# EUROPEAN PATENT APPLICATION

(11) **EP 4 317 458 A1**
(43) Date of publication of application: **07.02.2024**
(21) Application number: 22780994.4
(22) Date of filing: 29.03.2022
(51) Int. Cl.: C12Q 1/6886, G01N 33/574

(54) **FOLLICULAR THYROID CANCER-SPECIFIC MARKER**

(30) Priority: 30.03.2021 JP 2021057814
(71) Applicant: Nippon Medical School Foundation, Tokyo 113-8602 (JP); University Public Corporation Osaka, Osaka-shi, Osaka 545-0051 (JP)
(72) Inventor: JIKUZONO Tomoo, Tokyo 113-8602 (JP); ISHIBASHI Osamu, Sakai-shi, Osaka 599-8531 (JP)
(74) Representative: Altmann Stößel Dick Patentanwälte PartG mbB
(86) International application number: PCT/JP2022/015687
(87) International publication number: WO 2022/210783

(57) **Abstract**

A marker that can set a particularly clear cutoff value for distinguishing between follicular thyroid cancer (FTC) and follicular adenoma (FA), which is difficult in conventional pathological diagnosis is provided. Also provided are a method and a kit for diagnosing the development of FTC in a subject, comprising detecting expression of FAM19A2 in a sample derived from the thyroid tissue of the subject.

## Description

### Technical Field

The present invention provides a method for detecting follicular thyroid cancer in a subject, the method comprising detecting expression of FAM19A2 in a subject-derived sample, a test agent, and a kit therefor.

### Background Art

About 90% of thyroid cancer is papillary thyroid cancer, and others are follicular thyroid cancer (about 5%), undifferentiated cancer (about 1 to 2%), medullary cancer (about 1 to 2%), and thyroid malignant lymphoma (about 1 to 5%).

Papillary thyroid cancer (PTC), which accounts for the majority, can be diagnosed pathologically, including cytological examination, and excision surgery is performed when PTC is diagnosed. Meanwhile, follicular thyroid cancer (FTC) has almost the same cell morphology as follicular adenoma (FA), a benign tumor, and is known to be difficult to distinguish therefrom by conventional pathological methods. Even if the tumor is pathologically diagnosed as benign after surgery for tumor resection, there are more than a few cases in which distant metastasis to bone, lung, and the like is found after surgery and the tumor is clinically considered to be FTC. Periodic follow-up is performed after surgery, and if there is no distant metastasis for 10 years, it can be said that FA is definitely diagnosed based on the clinical course.

Patent Literature 1 proposes a combination of Trefoil Factor 3 (TFF3) mRNA and Galectin-3 (GLT3) mRNA, an internal control, as a thyroid tumor marker to be used to determine whether thyroid tumor in a specimen is benign tumor (FA) or follicular thyroid cancer (FTC), and describes that FTC can be determined when relative TFF3 mRNA expression is lower than the standard.

In Non Patent Literature 1, large-scale genome analysis and transcriptome analysis are performed by next-generation sequencing for thyroid tumors including FTC, and gene mutations in tumors are investigated. Non Patent Literature 2 reports that the expression of leucine rich-repeat kinase 2 (LRRK2), which is known as a molecule responsible for Parkinson's disease, is elevated in FTC patients as compared with normal tissues, and that it may be useful for diagnosing FTC.

The group of the inventors have already published a paper on a dataset of microarray analysis that is performed for cancerous and non-cancerous areas using formalin-fixed specimens surgically obtained from FTC patients (Non Patent Literature 3). The microarray (Clariom D, Thermo Fisher Scientific K.K.) used in this analysis is an ultra-high-density microarray loaded with 6 million or more probes and is useful for genome-wide transcriptome analysis such as alternative splicing analysis and long non-coding RNA analysis, in addition to general gene expression analysis. The dataset obtained by this analysis has been registered in Genomic Expression Archive or the like that is a public database of functional genomics data managed and operated by the National Institute of Genetics, and Non Patent Literature 3 is a commentary article on this dataset. Accordingly, Non Patent Literature 3 does not refer to the expression of specific genes or RNAs.

### Citation List

### Patent Literature

Patent Literature 1: Japanese Patent No. 4428932

### Non Patent Literature

Non Patent Literature 1: Nat. Commun. (2019) 2764
Non Patent Literature 2: Proc Natl Acad Sci USA. (2011) 25; 108
Non Patent Literature 3: BMC Res Notes (2020) 13: 241

### Summary of Invention

### Technical Problem

A definitive diagnosis of follicular thyroid cancer (FTC) and benign follicular adenoma (FA) is difficult and could not be made even in the pathological diagnosis after surgery. Only when no metastasis is developed after 10 years of follow-up after surgery, a definitive diagnosis of follicular adenoma (FA) can be made. If there is a distant metastasis, a definitive diagnosis of follicular thyroid cancer (FTC) can be made for the first time. This was a problem.

If the tumor is known to be benign before surgery, surgery is not necessarily required. However, since it is difficult to distinguish between FTC and FA (preoperative diagnosis), resection surgery is performed with consent only when there is some possibility that the tumor is malignant. Further, since a definite diagnosis cannot be made even after surgery, a follow-up is essential even if the postoperative pathological diagnosis indicates a benign tumor. This not only puts a heavy mental and economic burden on the patient but also puts a considerable burden on the medical staff.

In addition, it is impossible to distinguish between FTC and FA with the thyroid tumor markers reported so far. As a result of analyzing the expression of TFF3, which is currently considered to be one of the most relevant markers, the group of the inventors has found that TFF3 expression shows a tendency to decrease in FTC, but it is difficult to clearly identify FTC by TFF3.

Therefore, in order to distinguish between FTC and FA, there has been a need for a marker that enables us to set a particularly clear cutoff value.

### Solution to Problem

The inventors extracted RNA from the cancerous and non-cancerous regions of formalin-fixed, paraffin embedded tissues from three cases who developed distant metastasis after surgery and received a definitive diagnosis of FTC. Thereafter, they determined candidate genes that were differentially expressed in FTC by genome-wide transcript analysis using a high-performance microarray, and then conducted experiments to verify the validity of these candidates using an additional cohort including FA cases. As a result, FAM19A2 was found as an mRNA that is specifically elevated in FTC cases and is hardly elevated in benign FA cases. Further, they have also found that the detection of a specific region of FAM19A2 is particularly advantageous. These findings are consistent with the results of studies that were conducted using commercially available samples and transcriptome datasets of thyroid tumors registered in public databases. The present invention is achieved based on these findings.

That is, the present invention provides the following aspects:
1. A method for detecting follicular thyroid cancer (FTC) in a subject, the method comprising detecting expression of FAM19A2 in a biological sample derived from the subject.
2. The method according to 1 above, wherein the higher expression of FAM19A2 in the subject than a reference value indicates a possibility that the subject has FTC.
3. The method according to 1 or 2 above, wherein the expression of FAM19A2 is expression of FAM19A2 mRNA.
4. The method according to 1 or 2 above, wherein the expression of FAM19A2 is transcription into an mRNA (FAM19A2_Ex1-2) or mRNA precursor comprising exons 1 and 2 of the FAM19A2 gene.
5. The method according to 1 or 2 above, wherein the expression of FAM19A2 is expression of FAM19A2 protein.
6. The method according to any one of 1 to 5 above, comprising calculating the expression of FAM19A2 as an absolute value or a relative value, and comparing it with a cutoff value.
7. The method according to any one of 1 to 6 above, wherein the biological sample is thyroid tissue.
8. A test agent for showing subject's susceptibility to FTC, comprising a primer or probe, or antibody for detecting FAM19A2.
9. A kit for use in the method according to any one of 1 to 7 above, comprising: the test agent according to 8 above; and a reagent and/or a buffer for reaction.

The present invention also provides a method for diagnosing follicular thyroid cancer in a subject, the method comprising detecting FAM19A2 expression in a biological sample derived from the subject.

The present invention also provides a method for treating follicular thyroid cancer, the method comprising a step of diagnosing the presence of follicular thyroid cancer in a subject; and a step of administering a therapeutic agent to the subject.

This description includes the disclosure of Japanese Patent Application No. 2021-057814, which is the basis of the priority of this application.

### Advantageous Effects of Invention

The present invention provides a means for accurately distinguishing FTC, which is a malignant tumor requiring surgery, and FA, which is a benign tumor. The present invention provides better information on the need for surgery and follow-up and enables more reliable diagnosis.

### Brief Description of Drawings

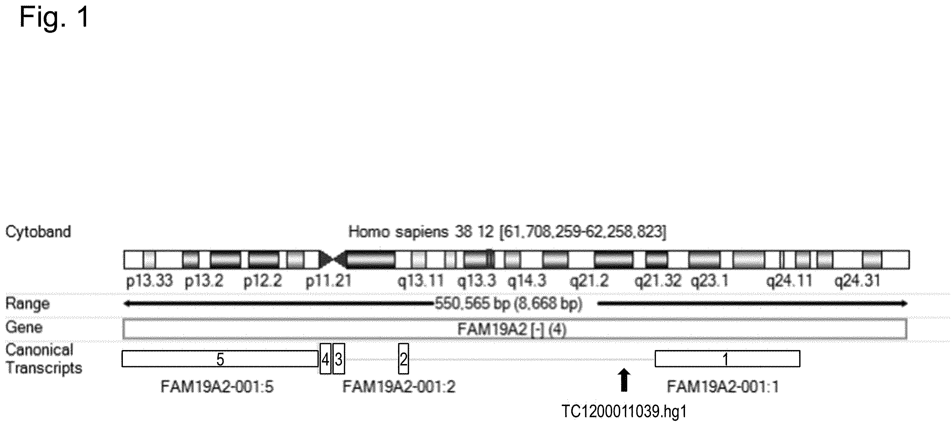

[Figure 1] Figure 1 schematically shows the FAM19A2 gene and the positions of exons on the chromosome. The sequence of RNA (TC1200011039.hg.1) found by the inventors is present at the position indicated by an arrow.

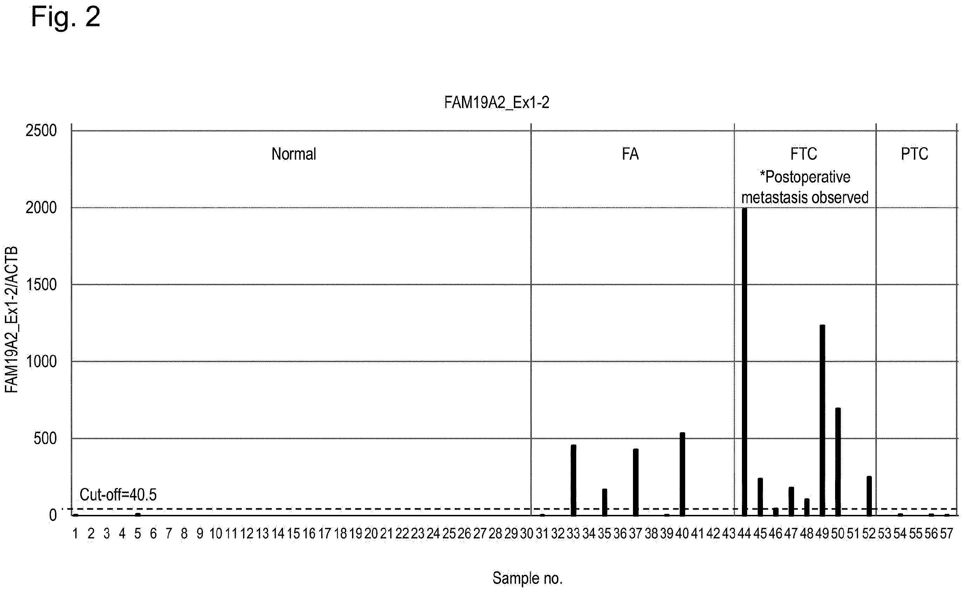

[Figure 2] Figure 2 shows the expression of FAM19A2_Ex1-2 in each patient in control group (Normal), follicular adenoma patient group (FA), follicular thyroid cancer patient group (FTC), and papillary thyroid cancer patient group (PTC). The vertical axis shows the expression of FAM19A2 relative to the expression of β-actin (ACTB).

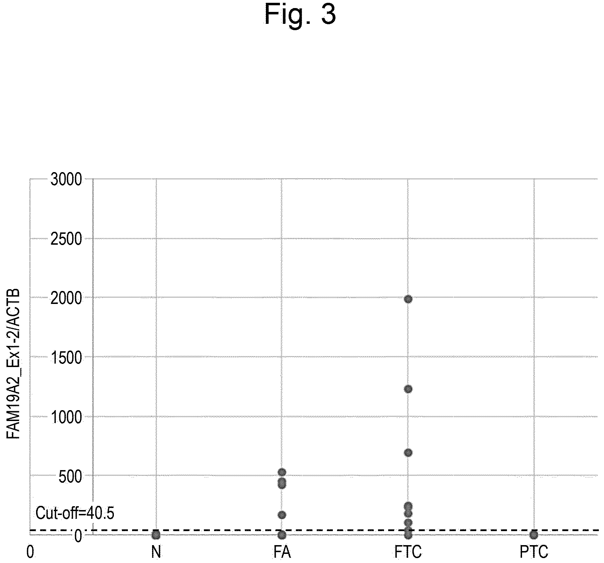

[Figure 3] Figure 3 is a scatter plot showing the expression of FAM19A2_Ex1-2 in control group (N), follicular adenoma patient group (FA), follicular thyroid cancer patient group (FTC), and papillary thyroid cancer patient group (PTC). The vertical axis shows the expression of FAM19A2 relative to the expression of β-actin.

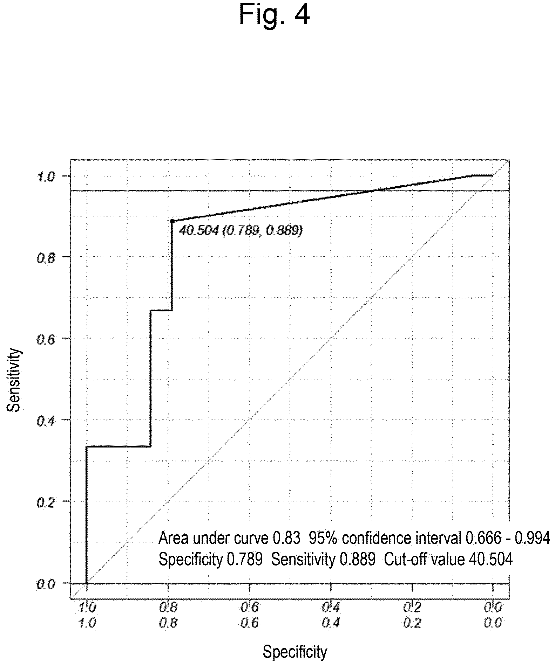

[Figure 4] Figure 4 shows an ROC curve plotted based on the expression of FAM19A2_Ex1-2 in follicular thyroid cancer tissue (FTC) and follicular adenoma tissue (FA).

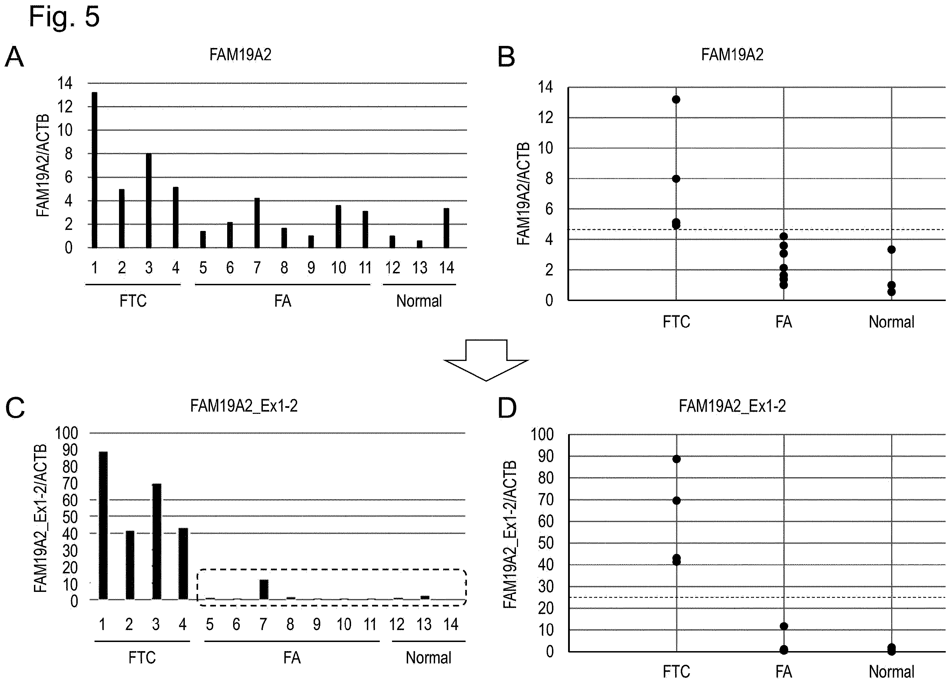

[Figure 5] Figure 5 shows the expressions of all splicing variants (A and B) of FAM19A2 mRNA and FAM19A2_Ex1-2 (C and D) in follicular thyroid cancer patient group (FTC), follicular adenoma patient group (FA), and normal tissue (Normal) relative to the expression of β-actin. B and D are scatter plots showing the expression values of A and C, respectively. 1: FTC total RNA (ORIGENE #559179); 2: FTC total RNA (ORIGENE #559636); 3: FTC total RNA (ORIGENE #562178); 4: FTC total RNA (ORIGENE #562190); 5: FA total RNA (ORIGENE #559146); 6: FA total RNA (ORIGENE #559956); 7: FA total RNA (ORIGENE #560640); 8: FA total RNA (ORIGENE #561376); 9: FA total RNA (ORIGENE #562016); 10: FA total RNA (ORIGENE #562170); 11: FA total RNA (ORIGENE #562832); 12: Normal thyroid RNA (Takara Bio Inc.); 13: Normal thyroid RNA (BioChain); 14: Normal thyroid RNA (Invitrogen). * Nos. 2 and 3 were derived from pulmonary metastatic tissue from the same patient.

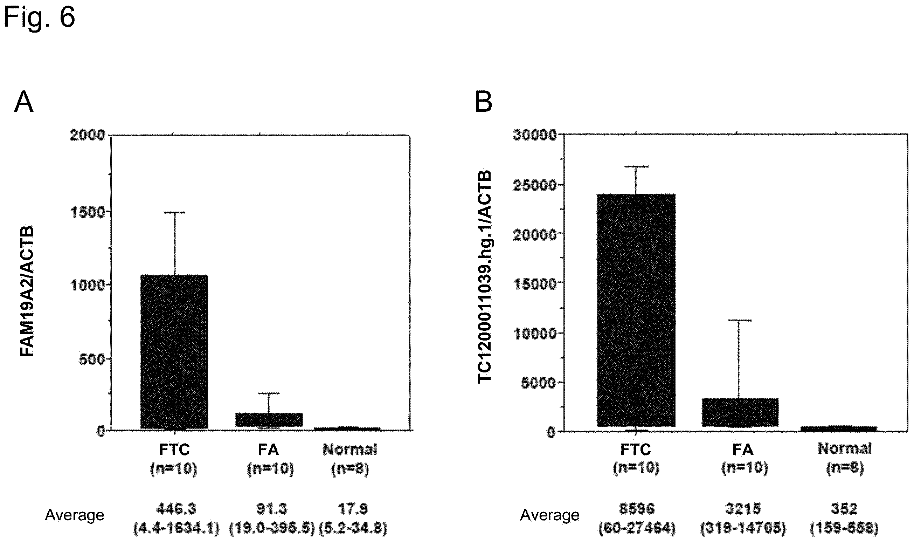

[Figure 6] Figure 6 shows the expressions of FAM19A2 (A) and TC1200011039.hg.1 (B) in follicular thyroid cancer tissue (FTC), follicular adenoma tissue (FA), and normal tissue (Normal) obtained from a thyroid tumor cohort of Kanagawa cancer center relative to the expression of β-actin.

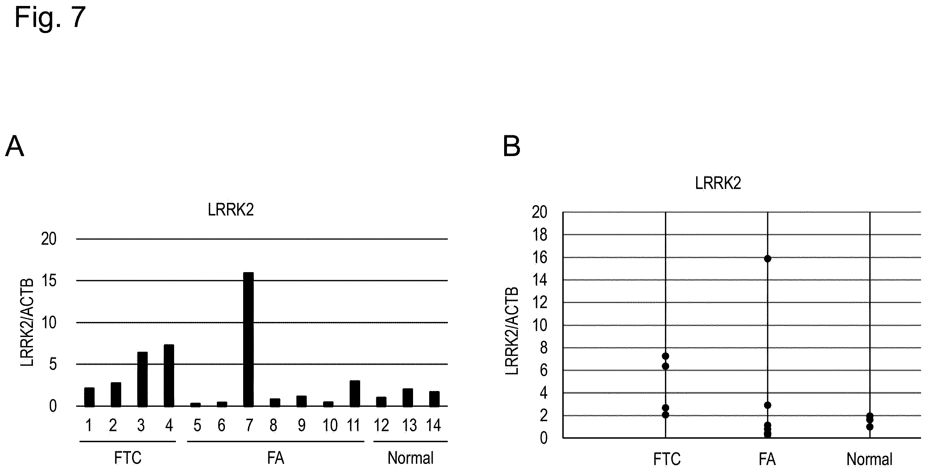

[Figure 7] Figure 7A shows the expression of LRRK2 in samples derived from follicular thyroid cancer patient group (FTC), follicular adenoma patient group (FA), and normal tissue (Normal) relative to the expression of β-actin. Figure 7B shows a scatter plot of the expression value in A. The same samples as those in Figure 5 were used.

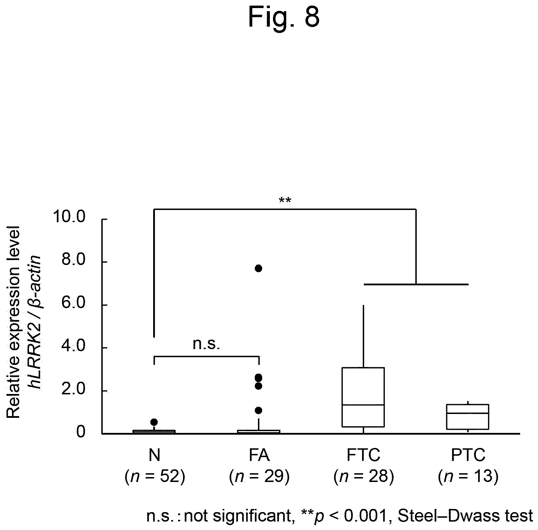

[Figure 8] Figure 8 is a boxplot showing the expression of LRRK2 mRNA in control group (N), follicular adenoma patient group (FA), follicular thyroid cancer patient group (FTC), and papillary thyroid cancer patient group (PTC). The vertical axis shows the expression of LRRK2 relative to the expression of β-actin.

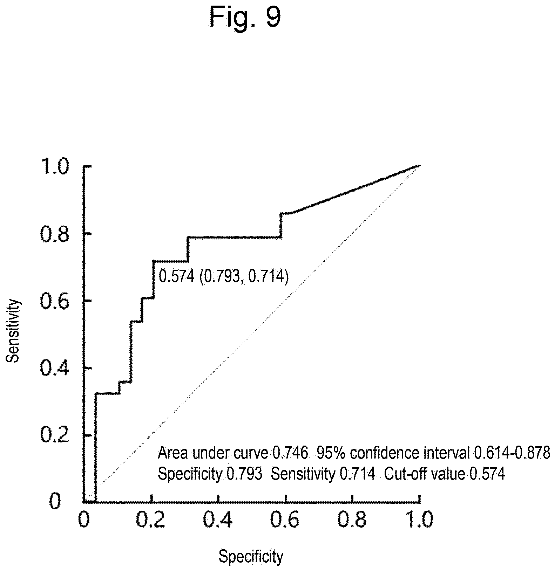

[Figure 9] Figure 9 shows an ROC curve plotted based on the expression of LRRK2 mRNA in follicular thyroid cancer tissue (FTC) and follicular adenoma tissue (FA).

### Description of Embodiments

### <Method for detecting follicular thyroid cancer>

The present invention provides a method for detecting FTC in a subject, the method comprising detecting FAM19A2 expression in a biological sample derived from the subject.

The inventors confirmed that the expression of FAM19A2 are remarkably elevated in FTC patients as compared with FA patients, PTC patients, and healthy subjects/normal tissues.

FAM19A2 is a protein also called Family with sequence similarity 19 (Chemokine (C-C motif)-like) member A2, TAFA2 (TAFA chemokine-like family member 2), or TAFA-2, and the gene encoding the protein is composed of 5 exons and introns therebetween. The amino acid sequence of the human FAM19A2 protein and the nucleotide sequence of the gene encoding the protein can be obtained, for example, from the database managed by the National Center for Biotechnology Information (NCBI) as Accession: AAH28403, Gene ID: 338811 or the like.

In the present invention, the subject is a human, or a non-human mammal such as a mouse, a rat, a rabbit, a pig, and a monkey, and is not specifically limited, and is preferably a human. Information on FAM19A2 in non-human mammals can also be obtained from the same databases as above or the like.

The subject may be a patient who has undergone a medical examination due to thyroid abnormality, or may be a subject who has undergone a physical examination, a complete medical examination, a cancer marker test, or the like. The subject may also be a patient before or after tumorectomy.

In the present invention, the biological sample is not specifically limited, and can be whole blood, blood plasma, serum, or thyroid tissue or cells collected from the subject. Preferably, the biological sample is thyroid tissue or cytodiagnosis specimen collected during tumorectomy or the like.

Accordingly, the method of the present invention can comprise a step of extracting RNA from thyroid tissue or cytodiagnosis specimen derived from a subject, for example, a formalin-fixed, paraffin-embedded (FFPE) specimen derived from cancer tissue and adjacent normal follicular tissue obtained from an FTC patient.

In the method of the present invention, the higher expression of FAM19A2 in a subject than a reference value indicates that the subject has FTC. Here, the "reference value" can be, for example, the value of the expression of FAM19A2 in a normal sample as an absolute value or relative value. In consideration that the expression of FAM19A2 is low in PTC patients and FA patients, the "reference value" can be the value of the expression of FAM19A2 in samples derived from PTC patients and FA patients, in addition to normal samples derived from healthy subjects or normal tissues. Alternatively, the "reference value" can be a cutoff value set based on the difference in the expression of FAM19A2 between FTC patients and FA patients.

For example, the values of FAM19A2 expression for a group of FTC patients and healthy subjects, optionally patients with diseases other than FTC (e.g., FA), or a control group including both are obtained from data derived from a large number of subjects. A cutoff value statistically obtained from these values, using multivariate analysis, logistic regression analysis, Receiver Operating Characteristic (ROC) curve, or the like, for example, can be used as a reference value. The reference value can be pre-set.

Accordingly, the method of the present invention can comprise a step of determining a cutoff value that can distinguish between FTC patients and FA patients, FTC patients and PTC patients, FTC patients and healthy subjects, or FTC patients and FA patients/PTC patients/healthy subjects.

Using the reference value, it is possible to predict the possibility that subjects are affected by FTC, based on the detection results from samples derived from the subjects. It is possible to provide data that the subjects are likely to have FTC, when the detection results of FAM19A2 derived from the subjects are high as compared with the reference value.

In an embodiment of the present invention, the expression of FAM19A2 to be detected is the expression of FAM19A2 mRNA. The expression of FAM19A2 mRNA can be detected, for example, by PCR reaction using appropriate primers or by hybridization using appropriate probes. The nucleotide sequences of the primers and probes can be appropriately determined based on the description herein and the common technical knowledge in the art, and can be synthesized based on the determined sequences. Primers and probes can be labeled as needed.

Accordingly, the method of the present invention can comprise a step of measuring the FAM19A2 mRNA expression by amplifying extracted RNA by qRT-PCR. More specifically, cDNA can be obtained by reverse transcription of extracted RNA and amplified by PCR reaction using a primer pair. Examples of the primer pair that can be used in the PCR reaction include, but not limited to, primer pairs that can amplify the sequence of ID: TC1200011039.hg.1 (SEQ ID No: 1) in Affymetrix Transcript Cluster (Affymetrix (currently, Thermo Fisher Scientific K.K.)) such as a primer pair having the sequences shown in SEQ ID Nos: 2 and 3 and a primer pair having the sequences shown in SEQ ID Nos: 4 and 5.

The method of the present invention can also detect FAM19A2 mRNA using a probe which can hybridize to the extracted RNA. The probe that can be used in the method of the present invention is not limited, and examples thereof include a probe having the sequence shown in SEQ ID No: 6.

According to the findings by the inventors, the expression of FAM19A2 can be confirmed by detection of the entire or any region of FAM19A2 mRNA. However, the inventors have also found that a particularly remarkable distinction can be made by detecting the transcription of mRNA or mRNA precursor comprising exons 1 and 2 of the FAM19A2 gene. Accordingly, in a preferable embodiment of the present invention, the detection of the expression of FAM19A2 to be detected can be detection of the mRNA or mRNA precursor comprising the exons 1 and 2 of the FAM19A2 gene, or the RNA sequence of intron 1 present between the exons 1 and 2.

In another embodiment of the present invention, the expression of FAM19A2 to be detected is the expression of FAM19A2 protein. The expression of FAM19A2 protein can be detected by an antigen-antibody binding reaction using antibodies or antigen-binding fragments thereof that specifically bind to the FAM19A2 protein.

Methods for generating antibodies when the antigen is specified are well known in the art. The antibodies that can be used in the present invention can be obtained as polyclonal antibodies by immunizing non-human mammals with the FAM19A2 protein or fragment thereof by known methods. Also, monoclonal antibodies can be obtained from hybridoma obtained by fusing antibody-producing cells that produce antibodies against the FAM19A2 protein with myeloma cells.

Antibodies can also be obtained by synthesis using genetic engineering techniques or chemical synthesis means based on information for the amino acid sequence of the antibody whose activity has been demonstrated or information for the nucleotide sequence of the polynucleotide encoding the antibody. Also, anti-FAM19A2 antibodies can be obtained from commercially available products for use.

The expression of FAM19A2 can be calculated as an absolute value or a relative value. In the case of calculating the expression of FAM19A2 as an absolute value, the amount of the entire or any region of FAM19A2 mRNA, mRNA or mRNA precursor comprising exons 1 and 2 of the FAM19A2 gene, RNA of the intron 1 present between the exons 1 and 2, or FAM19A2 protein or fragment thereof may be calculated as an amount per cell for example. In the case of calculating it as a relative value, the expression of FAM19A2 can be a relative value to the expression of other stably expressed proteins in FTC and control tissues, such as β-actin (ACTB) or glyceraldehyde 3-phosphate dehydrogenase (GAPDH).

Comparison of the calculated value with a pre-set cutoff value can provide useful information on whether or not the subject has FTC.

The cutoff value is not specifically limited, but could be calculated as a relative value to β-actin, in the case of the expression of mRNA comprising the exons 1 and 2 of FAM19A2 in samples of FTC tissue and FA tissue in Example 2 below, and set to about 40.5 from the Receiver Operating Characteristic (ROC) curve (Figure 4). In the future, it may be possible to obtain a more clinically accurate cutoff value, based on a larger number of cases. According to the findings by the inventors so far, the cutoff value can be calculated as a relative value to β-actin, for example, and set to a value in the range of 10 to 50, such as 15 to 45, 20 to 40, 25 to 35, 10, 11, 12, 13, 14, 15, 16, 17, 18, 19, 20, 25, 30, 35, 40, 45, and 50, but the cutoff value is not limited to the above. For example, in the case where the cutoff value was set to 10 in Example 2, almost the same results could be obtained as in the case where the cutoff value is set to 40.5.

Accordingly, the method of the present invention can comprise a step of comparing the expression of FAM19A2 in a biological sample derived from a subject with the cutoff value to determine whether or not the expression of FAM19A2 in the subject exceeds the cutoff value set above, or the expression level is elevated as compared with that in healthy subjects.

The inventors also have found by analysis of Example 1 that the expression of LRRK2 mRNA is also elevated in FTC. This was also confirmed by analyses using commercially available samples and transcriptome datasets of thyroid tumors registered in public databases, and similar results have been reported by other groups (Clin Cancer Res (2008); 14 (11)). This confirms the validity of the samples and datasets used by the inventors.

In addition, the above findings suggest that the susceptibility to follicular thyroid cancer can be predicted using the expression of LRRK2 as an index. In other words, like FAM19A2 expression, LRRK2 expression was also confirmed to be elevated in follicular thyroid cancer tissues compared to healthy subjects/normal tissues. The detection results of LRRK2 can also be calculated as absolute or relative values in the same way as the detection results of FAM19A2 and compared with the cutoff value to indicate the susceptibility to follicular thyroid cancer.

The amino acid sequence of the human LRRK2 (leucine-rich repeat kinase 2) protein and the nucleotide sequence of the gene encoding this protein can be obtained, for example, from the database managed by NCBI as Accession: NP_940980, Gene ID: 120892 or the like.

Accordingly, the method of the present invention can combine the detection results for the expression of FAM19A2 with the detection results for the expression of LRRK2.

More specifically, the expressions of FAM19A2 and LRRK2 in a subject are each measured, and in the case that the expression of FAM19A2 is elevated as compared with the cutoff value determined in advance, and also the expression of LRRK2 is elevated as compared with the cutoff value determined in advance, it is indicated that there is a possibility that the subject has FTC.

When the possibility of FTC in the subject is indicated by the method of the present invention, the subject can be diagnosed by a doctor as to whether or not the subject has FTC.

### <Diagnosis method>

The present invention also provides a method for diagnosing follicular thyroid cancer in a subject, which is characterized by detecting the expression of FAM19A2 in a biological sample derived from the subject.

### <Treatment method >

The present invention further provides a method for treating follicular thyroid cancer, comprising a step of detecting expression of FAM19A2 in a biological sample derived from a subject and a step of administering a therapeutic agent to the subject.

The treatment method for follicular thyroid cancer is surgery as the first choice, followed by internal radioactive iodine therapy when distant metastasis is observed during postoperative follow-up, but treatment using molecular-targeted drugs or the like is also possible. In consideration of the conditions of the patient and diseases, it is also possible to perform treatment, for which surgery, radioactive iodine, molecular-targeted drugs, and the like, are appropriately combined.

Examples of agents that can be used for treating follicular thyroid cancer include specific monoclonal antibodies against the protein encoded by FAM19A2 (FAM19A2 protein).

There are very few reports on the function of FAM19A2 protein, and the involvement of FAM19A2 protein in the progression and metastasis of follicular thyroid cancer is unknown. However, the FAM19A2 protein is a chemokine-like protein, and antibodies against this protein may suppress the progression and metastasis of follicular thyroid cancer. In particular, FAM19A2_Ex1-2, which is highly expressed specifically in follicular thyroid cancer, is the only one known splicing variant of FAM19A2 that encodes a protein, and antibodies against this protein are expected to have extremely high specificity. In fact, a specific antibody against the FAM19A5 protein, which is highly homologous to the FAM19A2 protein, has been reported as a technique that can be applied to the diagnosis and treatment of brain tumors (Japanese Patent Publication (Kokai) No. 2021-185151).

### <Test agent/kit>

The present invention also provides a test agent that indicates a subject's susceptibility to FTC, comprising primers or probes, or antibodies for detecting FAM19A2.

As described above, the expression of FAM19A2 in a subject can be detected by detecting FAM19A2 mRNA or FAM19A2 protein in a biological sample derived from a subject.

When intended to detect FAM19A2 mRNA, the test agent of the present invention may comprise primers capable of detecting FAM19A2 mRNA by PCR or probes capable of detecting FAM19A2 mRNA by hybridization. The nucleotide sequences of the primers and probes can be appropriately determined based on the description herein and common technical knowledge in the art. Primers and probes can be labeled as needed. The probes can also be used as a part of a DNA array together with probes used for the detection of other mRNAs.

When intended to detect FAM19A2 protein, the test agent of the present invention may comprise antibodies or antigen-binding fragments thereof that specifically bind to the FAM19A2 protein. Antibodies or antigen-binding fragments thereof can be labeled as needed.

In addition to the test agent of the present invention, the present invention also provides a kit to be used for the method of the present invention, the kit comprising instructions for use with information including reagents, buffers, and reference values required for the reaction. The kit can appropriately contain reagents necessary for hybridization, nucleotides necessary for amplification reaction, optionally labeled secondary antibodies, and the like.

When detecting the expression of LRRK2 as well in combination with the detection of the expression of FAM19A2, the kit can further contain primers or probes for detection of LRRK2 or anti-LRRK2 antibodies.

### Examples

Hereinafter, the present invention will be described further in detail by way of examples, but the present invention is not limited to these examples.

### [Example 1: Transcripts whose expression is elevated in FTC]

Formalin-fixed, paraffin-embedded (FFPE) specimens from cancer tissue and adjacent normal follicular tissue obtained from 3 FTC patients were used for comparative expression analysis using the similar method as BMC Res Notes (2020) 13:241.

Specifically, each FFPE specimen was cut into 20 µm sections, RNA was extracted using NucleoSpin^{®} totalRNA FFPE XS (Takara Bio Inc.), and genome-wide transcriptome analysis was performed using the Affymetrix Clariom D Assay (Thermo Fisher Scientific K.K.), which is the next-generation microarray for transcriptome profiling.

As a result, the expression (transcription) was 49.5-fold higher in FTC tissue than in normal tissue, and an RNA whose corresponding gene was unknown was found as the transcript with the highest increase in expression. This RNA corresponds to ID: TC1200011039.hg.1 in the Affymetrix Transcript Cluster (Affymetrix (currently, Thermo Fisher Scientific K.K.)).

The nucleotide sequence of this RNA is shown below along with the positions of suitable primers used for its PCR:
TC1200011039.hg.1:TTTGTACTTCAGGTGCACAGAATTGGATTAATAATAATCCGTG GCTTATAACATACTCTCAGCATTGGTAAT (SEQ ID No: 1)
Fw:GTACTTCAGGTGCACAGAAT (SEQ ID No:2)
Rv:ATACTCTCAGCATTGGTAAT (SEQ ID No:3)

Since the aforementioned RNA is derived from the sequence in the intron present between exons 1 and 2 of the mRNA encoding FAM19A2, it was considered that it may be a non-coding RNA that does not encode a protein or a precursor mRNA of FAM19A2 before spliced. Figure 1 schematically shows the positions of the FAM19A2 gene and exons on the first chromosome. The sequence of the aforementioned TC1200011039.hg.1 exists at the position indicated by an arrow.

Therefore, it was suggested that the unidentified RNA and FAM19A2 could be biomarkers for follicular thyroid cancer.

Furthermore, in this example, LRRK2 mRNA was found as a transcript showing 17.9-fold higher expression (transcription) in FTC tissue than in normal tissue, with the third highest increase in expression. This result is consistent with reports by other groups of upregulation of LRRK2 in FTC tissues (Clin Cancer Res (2008);14(11)).

### [Example 2: Analysis of mRNA expression level of FAM19A2_Ex1-2 in FTC]

It is often difficult to morphologically distinguish FTC, especially minimally invasive FTC, from FA, and the possibility of incorrect diagnosis cannot be ruled out in analyses using fresh specimens of surgically resected follicular tumor tissue. Meanwhile, follow-up information after surgery can be obtained if pathological specimens preserved after formalin-fixed, paraffin-embedding (hereinafter referred to as FFPE) is used; therefore, it is possible to select and analyze only cases with distant metastasis, that is, cases that are extremely likely to be FTC.

In this example, RNA was extracted from FFPE tissues of patients who were diagnosed with FTC at the time of initial surgery and who had distant metastases during the postoperative follow-up period, who were diagnosed with FA, and who were diagnosed with PTC, and the mRNA expression level of FAM19A2_Ex1-2 was analyzed by TaqMan qRT-PCR. The sequences of the Fw primer, Rv primer and FAM-TAMRA fluorescence-labeled probe used in this analysis are shown below.
Fw: 5'-AGGAGCATCGCTAGGTGTTG-3' (SEQ ID No: 4)
Rv: 5'-ATCTCTTACTCATCCTGCAGCC-3' (SEQ ID No: 5)
Probe: 5'-FAM-CGCCACCGGGAAGCG-TAMRA-3' (SEQ ID No: 6)

For comparison, RNA was similarly extracted from normal follicular tissue adjacent to each tumor tissue and subjected to analysis. As an internal standard, ACTB mRNA expression level was also analyzed in the same manner. TaqMan Gene Expression Assays Hs99999903_m1 (manufactured by Thermo Fisher Scientific K.K.) was used for ACTB analysis.

The expression level of FAM19A2_Ex1-2 was shown as a relative value of the corrected value using the expression level of ACTB mRNA as an internal standard. At that time, the corrected value for one case of normal thyroid follicular tissue was set to 1 for convenience.

As a result, as shown in Figures 2 and 3, when the cutoff value was set to 40.5 for example, the expression level of FAM19A2_Ex1-2 exceeded the cutoff value in 8 of 9 cases in FTC tissues (89%). Meanwhile, exceedance of the cutoff value was shown only in 4 of 13 FA cases (31%). In PTC cases, all of 5 analyzed cases were below the cutoff value.

Based on the results obtained in this example, the expression level of FAM19A2_Ex1-2 in FTC and FA tissues was set as an explanatory variable, and FA or FTC was set as the objective variable as a binary categorical variable to plot an ROC curve (Figure 4).

As a result, the area under the curve (AUC) and 95% confidence interval were calculated to be 0.83 and 0.666 to 0.994, respectively. When the expression cutoff value was set to 40.504, the sensitivity and specificity were 0.889 and 0.789, respectively. Therefore, it was shown that the expression level of FAM19A2_Ex1-2 is effective in distinction between the two tumors.

Another ROC curve based on normal tissue and FTC tissue data was also plotted in the same way, and it was confirmed that they could be clearly distinguished (AUC = 0.937, sensitivity = 0.889, specificity = 1.000). However, since it is clinically possible to distinguish between normal tissue and FTC or FA tissue, the distinction between FA and FTC as described above is more important.

### [Example 3: Analysis of FAM19A2 mRNA expression level in FTC 1]

Using commercially available total RNA samples extracted from thyroid follicular tumor tissue (three examples of FTC and seven examples of FA) and normal thyroid follicular tissue (four examples) (thyroid follicular tumor tissues were obtained from OriGene Technologies, Inc., normal thyroid follicular tissues were obtained from BioChain Institute Inc., Takara Bio Inc., and Thermo Fisher Scientific K.K.), the expression level of FAM19A2_Ex1-2 mRNA in each tissue was measured using the TaqMan PCR method.

First, 1 µg of RNA for each sample was reverse transcribed in 20 µl of reverse transcription solution to prepare cDNA. PrimeScript^{™} RT reagent Kit (Perfect Real Time) (manufactured by Takara Bio Inc.) was used for this reverse transcription reaction, and the reaction conditions were set according to the manufacturer's instructions.

Next, the cDNA was diluted 16-fold with RNase-free sterile distilled water, and 2 µl of the diluted solution was used to semi-quantitatively analyze the expression level of human FAM19A2_Ex1-2 by TaqMan qPCR method. Similarly, the expression level of human ACTB (β-actin gene) mRNA was semi-quantitatively analyzed.

The relative expression level of FAM19A2_Ex1-2 mRNA can be calculated using a housekeeping gene commonly used for gene expression correction such as ACTB or GAPDH mRNA as an internal standard by the 2^{-ΔΔCt} method. In this example, the relative expression level of FAM19A2_Ex1-2 mRNA was calculated using ACTB mRNA as an internal standard. The following reagents were used as primers and probes that recognize these mRNAs.

Human FAM19A2_Ex1-2: TaqMan Gene Expression Assays Hs06596816_m1 (Thermo Fisher Scientific K.K.)
Human ACTB: TaqMan Gene Expression Assays Hs99999903_m1 (Thermo Fisher Scientific K.K.)

For comparison, the expression levels of all currently known FAM19A2 mRNA splicing variants were also analyzed by TaqMan qPCR. At this time, TaqMan Gene Expression Assays Hs00698713_m1 (Thermo Fisher Scientific K.K.) were used as primers and probes.

As a result, as shown in Figure 5, the expression of all splicing variants of FAM19A2 was low in FA and normal tissue (Normal), and the relative expression value to ACTB was about 4.5 or less, compared to 5 or more in FTC, indicating that FTC can be clearly distinguished from FA and normal.

Furthermore, the expression of FAM19A2_Ex1-2 mRNA showed that a relative expression value to ACTB was about 12 or less in FA and normal tissues (Normal), compared to over 40 in FTC, indicating that clearer distinction is possible by setting a highly reliable cutoff value.

### [Example 4: Analysis of FAM19A2 mRNA expression level in FTC 2]

Using the RNA obtained from the thyroid tissue of the thyroid tumor cohort collected at Kanagawa cancer center, with the support of the "Platform of Supporting Cohort Study and Biospecimen Analysis" (http://cohort.umin.jp/) of the Ministry of Education, Culture, Sports, Science and Technology Research Grant, expression level analysis was performed in the same manner as in Example 3.

As a result, as shown in Figure 6, both the expression of FAM19A2 (A) and the expression of TC1200011039.hg.1 (B) were confirmed to be significantly elevated in FTC as compared with FA and normal tissues.

### [Example 5: Analysis of FAM19A2 mRNA expression level in FTC 3]

Focusing on the expression of FAM19A2_Ex1-2 among FAM19A2 mRNAs, it was investigated whether the expression in FTC was significantly higher than in benign tumor (FA).

Using NGS data for tumor tissue and paired normal follicular tissue in each case of follicular thyroid cancer (FTC), follicular adenoma (FA), follicular papillary thyroid cancer of the PTC subtype (PTC-FV), papillary thyroid cancer (PTC) in the dataset (SRA accession No. ERP012979) available from NCBI's Sequence Read Archive (SRA) database (https://trace.ncbi.nlm.nih.gov/Traces/sra/?study=ERP012979), a comparative analysis of the expression level of FAM19A2_Ex1-2 was performed.

Transcripts Per Million (TPM) was used as an index of expression level. The TPM was derived according to the following procedure. First, the above data was downloaded in SRA format and then converted to FASTQ format by fastq-dump. The obtained FASTQ files were quality-checked by FastQC, and then mapped to the human genome sequence using hisat2. At this time, NCBI's GRCh38 was used as the reference genome sequence.

After converting and sorting the SAM format data obtained by mapping into BAM format using the samtools function, stringtie was used to create GTF files containing gene expression information. Referring to the GTF files, information on the TPM of the gene of interest, namely FAM19A2_Ex1-2 (the corrected value obtained by dividing the total number of reads per 1 kb of transcript by 10⁶) was obtained. For comparison, the sum of TPMs of all currently known FAM19A2 mRNA splicing variants (isoforms) was similarly derived.

The results of expressions of all isoforms of FAM19A2 or FAM19A2_Ex1-2 in normal and FTC tissues, and expressions of all isoforms of FAM19A2 or FAM19A2_Ex1-2 in normal and FA tissues (TPM) are shown in Tables 1 and 2, respectively. As shown in Table 1, FAM19A2_Ex1-2 is rarely expressed in normal tissues, whereas all isoforms are highly expressed in FTC tissues and among them FAM19A2_Ex1-2 is expressed in a large proportion. As a result, the T/N ratio (FTC/N) is significantly higher when the expression of FAM19A2_Ex1-2 is detected than when the expression of all isoforms is detected. Meanwhile, as shown in Table 2, the expression of FAM19A2_Ex1-2 at a high proportion, which was shown in FTC, was not shown in FA tissues, although the expressions of all isoforms of FAM19A2 and FAM19A2_Ex1-2 were shown to be elevated in FA tissue compared to normal tissue.

**[Table 1]**

| FAM19A2 isoform | TPM | | Fold change (FTC/N) |
|---|---|---|---|
| | Normal | FTC | |
| All | 1.602 | 12.93 | 8.071 |
| FAM19A2_EX1-2 | 0.2381 | 8.933 | 37.52 |

**[Table 2]**

| FAM19A2 isoform | TPM | | Fold change (FA/N) |
|---|---|---|---|
| | Normal | FA | |
| All | 1.882 | 4.009 | 2.130 |
| FAM19A2_EX1-2 | 0.2619 | 1.834 | 7.003 |

Next, when comparing the expression level of FAM19A2_Ex1-2 mRNA in each tumor and its paired normal tissue, the TPM value of FAM19A2_Ex1-2 in normal thyroid tissue was less than 10 in all cases, but the TPM value in FTC tissue was 10 or more in 7 of 19 cases (37%). Meanwhile, in FA, only 1 of 18 cases (5.7%) showed a TPM of 10 or higher. In PTC, only 2 of 27 cases (7.4%) showed a TPM of 10 or higher. Table 3 shows the breakdown of the number of cases of each thyroid tumor and the results.

From the above, FAM19A2_Ex1-2 mRNA expression tended to be higher in tumor tissues diagnosed as FTC than in tumor tissues diagnosed as FA or PTC.

**[Table 3]**

| Thyroid tissue | Number of cases | Number of cases with TPM > 10 |
|---|---|---|
| Follicular adenoma | 18 | 1 (5.7%) |
| Follicular thyroid cancer | 19 | 7 (37%) |
| Follicular papillary thyroid cancer | 11 | 2 (18%) |
| Papillary thyroid cancer | 27 | 2 (7.4%) |

### [Example 6: Analysis of LRRK2 expression level in FTC]

As a result of the array analysis in Example 1, the inventors have found that the expression of LRRK2 mRNA is also enhanced in FTC, and this result is consistent with reports from other groups.

In this example, the expression of LRRK2 was measured using commercially available RNA samples derived from FTC tissue, FA tissue, and normal thyroid tissue used in Example 3, and compared with the expression of FAM19A2_Ex1-2 analyzed in Example 3.

The expression level of LRRK2 was calculated as a relative value to the expression of ACTB using TaqMan Gene Expression Assays Hs01115057_m1 (manufactured by Thermo Fisher Scientific K.K.).

As a result, as shown in Figure 7, the expression of LRRK2 also tended to increase in FTC tissues compared to normal tissues and FA tissues except for one case, but it was confirmed that the increase in expression of FAM19A2_Ex1-2 shown in Figure 5 was more pronounced and stable. Therefore, it was demonstrated that FAM19A2_Ex1-2 was superior to LRRK2 as a biomarker for follicular thyroid cancer.

### [Example 7: Analysis of mRNA expression level of LRRK2 in FTC]

The expression of LRRK2 was measured and analyzed using RNA obtained from FFPE samples of FTC tissue (n=28), FA tissue (n=29), PTC tissue (n=13), and normal thyroid tissue (n=52).

The expression of LRRK2 was analyzed in the same manner as in Example 6. As an internal standard, the expression of ACTB mRNA was analyzed in the same manner as in Example 2, and the expression level of LRRK2 was calculated as a relative value to the expression level of ACTB.

As a result, as shown in Figure 8, the expression of LRRK2 tended to increase in FTC tissue as compared with normal tissue and FA tissue.

In the case where the cutoff value was set to 0.574, the expression level of LRRK2 exceeded the cutoff value in FTC tissues in 20 of 28 cases (71.4%). Meanwhile, only 6 of 29 (20.7%) FA cases exceeded the cutoff value. The expression of LRRK2 tended to increase in PTC cases (13 cases) as well. The expression of LRRK2 tended to increase in PTC cases (13 cases) as well.

As a result of creating the ROC curve based on the results obtained in this example, and setting the expression levels of LRRK2 in FTC and FA tissues as explanatory variables and the target variable as a binary categorical variable to FA or FTC, the area under the curve (AUC) and the 95% confidence interval were calculated to be 0.746 and 0.614 to 0.878, respectively, in the comparison between FTC and FA (Figure 9). The specificity and sensitivity were 0.793 and 0.714, respectively, when the cutoff value of expression level was set to 0.574. Accordingly, the expression level of LRRK2 was also shown to be effective in distinguishing between FTC and FA.

Meanwhile, in PTC, the expression of FAM19A2_Ex1-2 did not increase (Figures 2 and 3), but the expression of LRRK2 tended to increase. Therefore, FAM19A2_Ex1-2 was superior to LRRK2 in terms of FTC-specific expression. However, the results of this example suggest the possibility of developing better biomarkers by combining F AM19A2 _Ex 1-2 and LRRK2.

### Industrial Applicability

When thyroid cancer is suspected, fine-needle aspiration cytology is currently performed as a pathological examination using a syringe-needle-sized needle; however, this cannot be said to be useful in diagnosing FTC. Use of the present invention may provide higher diagnostic value than cytological diagnosis in cases other than those clearly diagnosed as PTC (accounting for 90% of the total).

Currently, joint development of a new cancer gene panel test device using RNA is underway, and it is expected that the biomarker RNA in the present invention will be incorporated into such a panel as a new test item.

All publications, patents and patent applications cited herein are hereby incorporated by reference in their entirety.

## Claims

1. A method for detecting follicular thyroid cancer (FTC) in a subject, the method comprising detecting expression of FAM19A2 in a biological sample derived from the subject.

2. The method according to claim 1, wherein the higher expression of FAM19A2 in the subject than a reference value indicates a possibility that the subject has FTC.

3. The method according to claim 1 or 2, wherein the expression of FAM19A2 is expression of FAM19A2 mRNA.

4. The method according to claim 1 or 2, wherein the expression of FAM19A2 is transcription into an mRNA or mRNA precursor comprising exons 1 and 2 of the FAM19A2 gene.

5. The method according to claim 1 or 2, wherein the expression of FAM19A2 is expression of FAM19A2 protein.

6. The method according to any one of claims 1 to 5, comprising calculating the expression of FAM19A2 as an absolute value or a relative value, and comparing it with a cutoff value.

7. The method according to any one of claims 1 to 6, wherein the biological sample is thyroid tissue.

8. A test agent for showing subject's susceptibility to FTC, comprising a primer or probe, or antibody for detecting FAM19A2.

9. A kit for use in the method according to any one of claims 1 to 7, comprising: the test agent according to claim 8; and a reagent and/or a buffer for reaction.
